# EUROPEAN PATENT SPECIFICATION

(11) **EP 0 548 191 B1**
(45) Date of publication and mention of the grant of the patent: **29.10.1997**
(21) Application number: 91916462.4
(22) Date of filing: 12.09.1991
(51) Int. Cl.: C07K 1/00, C07K 2/00

(54) **SEPARATION OF PROTEINS AND DYES**
TRENNUNG DER PROTEINE UND FARBSTOFFE
SEPARATION DE PROTEINES ET DE COLORANTS

(30) Priority: 12.09.1990 GB 9019919
(43) Date of publication of application: 30.06.1993
(73) Proprietor: Delta Biotechnology Limited, Nottingham NG7 1FD (GB)
(72) Inventor: JOHNSON, R. A. Delta Biotechnology Limited, Nottingham NG7 1FD FD (GB); QUIRK, Alan, Victor Delta Biotechnology Limited, Nottingham NG7 1FD (GB); WOODROW, John, Rodney Delta Biotechnology Limited, Nottingham NG7 1FD (GB)
(74) Representative: Bassett, Richard Simon
(86) International application number: GB9101556
(87) International publication number: WO9204367

(56) References cited:
- EP-A- 0 395 896
- EP-A- 0 464 590
- DE-A- 2 342 058
- GB-A- 2 053 926
- Protein Purification, 2nd edition, 1988, pages 141-156, Springer-Verlag, New York, US; Robert K. Scopes
- JOURNAL OF CHROMATOGRAPHY, vol. 57, 1971, Amsterdam, NL; M.-P. VAN DAMME et al, pages 158-160
- TRANSFUSION, vol. 27, 1987; M.-J. KING: "Hemagglutination enhancement by bovine serum albumin is affected by octanoate, reactive blue 2 (Cibacron Blue), and polymer", pages 302-308
- CHEMICAL ABSTRACTS, vol. 95, 1981, page 205, abstract no. 127700v, Columbus, Ohio, US; U. KRAGH-HANSEN: "Effects of aliphatic fatty acids on the binding of Phenol Red to human serum albumin", & BIOCHEM. J. 1981, 195(3), 603-613

## Description

The present invention relates to the purification of proteins. In this specification, the term "protein" includes naturally-occurring proteins, non-naturally-occurring proteins and other polypeptides which are large enough to have a ligand binding site, and the term "purification" means "rendering more pure", rather than conferring a given level of purity.

In the separation of proteins from natural sources or, particularly, from the media of fermentations in which a genetically engineered host cell produces the protein, a protein-containing liquid is often passed through a chromatographic column consisting of a protein-binding compound bound to a solid support. The protein-binding compound binds to a ligand-binding site on the protein whilst the other material passes through the column and the protein is later eluted from the column in a purer form.

However, a small proportion of the protein-binding compound and/or a portion thereof sometimes elutes with the protein and must later be separated from the protein, particularly if the protein is intended for medical use. There have been prior proposals simply to absorb the dye onto a column of cross-linked Sephadex (R.T.M., Pharmacia).

Scopes, R.K., in "Protein Purification, Principles and Practice" (Springer Verlag, N.Y., USA, 2nd Edition, pp 141-157), mentioned that trace amounts of dye in the eluate from dye-containing columns can be removed on anion exchangers but did not disclose whether it was the protein or the dye which should bind to the anion exchanger and did not mention the use of a disrupting agent. GB-A-2 053 926 disclosed the use of, amongst other things, a buffer containing sodium chloride and sodium caprylate to elute human serum albumin from an affinity medium. However, what those in the art would then have done, whether or not a dye contamination problem was perceived, was to dialyse away the salt and caprylate before further treatment. What we have now found is that combining the anion exchanger process with the use of a high salt/caprylate concentration to disrupt the dye-protein binding allows efficient separation of the dye from the desired protein.

Accordingly, one aspect of the present invention provides a process for removing some or all of a protein-binding compound from an aqueous liquid containing the protein-binding compound bound to a protein, the process comprising the steps of (1) exposing the liquid to a disrupting material comprising a mixture of a salt and a compound which disrupts hydrophobic interactions between the protein and the protein-binding compound, (2) exposing the liquid from step (1) to an ion exchange resin to bind the protein-binding compound to the resin and (3) separating the resin to yield the protein, wherein either (i) the concentration of the salt is at least 0.5M, or (ii) the protein-binding compound is a synthetic textile dye or a protein-binding intermediate or derivative thereof, or (iii) the protein is human albumin.

A second aspect provides a process for removing some or all of a protein-binding compound from an aqueous liquid containing the protein-binding compound bound to a protein, the process comprising the steps of (1) exposing the liquid to an ion exchange resin to bind the protein-binding compound to the resin, (2) contacting the thus-bound protein-binding compound with a disrupting material comprising a mixture of a salt and a compound which disrupts hydrophobic interactions between the protein and the protein-binding compound, and (3) separating the resin to yield the protein, wherein either (i) the concentration of the salt is at least 0.5M, or (ii) the protein-binding compound is a synthetic textile dye or a protein-binding intermediate or derivative thereof, or (iii) the protein is human albumin.

The step involving the disrupting material and the step of binding the protein-binding compound to the resin may be simultaneous or may overlap such that the liquid is still exposed to the disrupting agent at the time that it is exposed to the resin. Step (3) is usually performed by passing the liquid through a column of the resin such that a solution of the protein, relatively free of the protein-binding material, is obtained.

The process is particularly well suited to removing synthetic textile dye compounds of the sort which have been disclosed in the literature for purifying proteins. Many such proteins (probably thousands) can be purified by the use of such dyes. To pick just one dye, Cibacron Blue 3-GA, this can be used to purify kinases, dehydrogenases and most other enzymes requiring adenyl-containing co-factors, for example NADP⁺ and NAD⁺. Such proteins include alcohol dehydrogenase, adenylate cyclase, adenylate kinase, glucose-6-phosphate dehydrogenase, hexokinase, phosphofructokinase and glyceraldehyde-3-phosphate dehydrogenase. Although the Cibacron Blue 3-GA dye will bind to these classes of proteins, it is also possible to use the Cibacron Blue 3-GA dye to purify proteins that do not have the dinucleotide binding site. These include albumin, lipoproteins, blood coagulation factors, interferon and thyroxin binding globulin. These dye compounds are usually anionic, in which case an anion-exchanger is most appropriate in the process of the invention, but some are cationic, in which case a cation-exchanger is most appropriate. The protein-binding compound is preferably a polysulphonated aromatic compound and is most preferably a triazine dye. Procion Brown MX-5BR, Cibacron Blue 3-GA, (suitable for separating human serum albumin), Procion Red H-8BN (for carboxypeptidase G2), Procion Yellow MX-AG (for IMP dehydrogenase), Procion Red HE-3B (for lactate dehydrogenase), Procion Green H-4G (for hexokinase), Procion Blue MX-4GD (for malate dehydrogenase), Procion Red H-3B (for 3-hydroxybutyrate dehydrogenase) and Procion Blue MX-R (for L-lactate dehydrogenase) are examples. These and others are summarised in the following table:

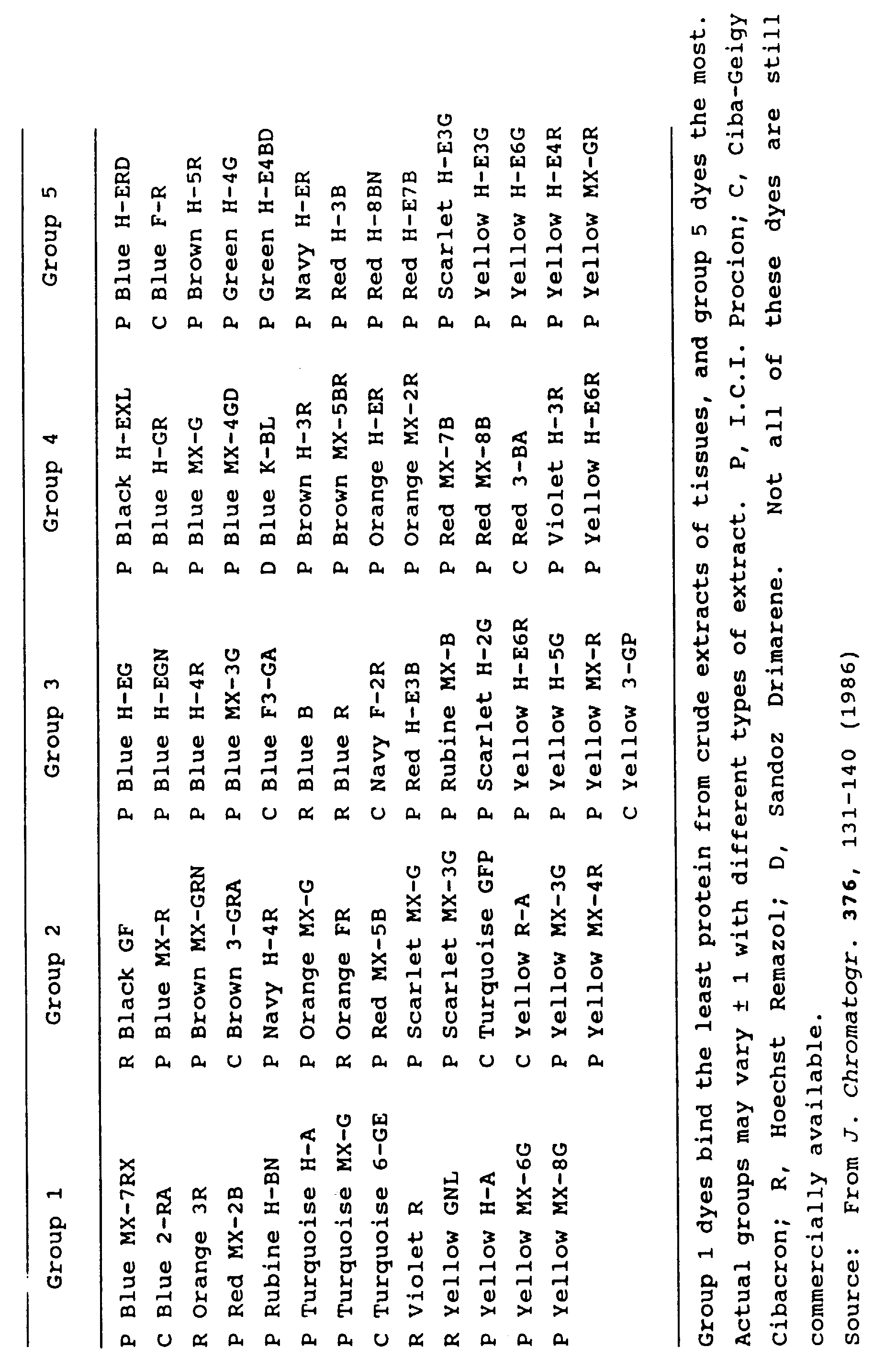

The dye itself (with or without the spacer which is commonly used to attach the dye to a column) may cause the contamination, or the problem may be caused by a derivative of the dye or an intermediate used in the synthesis of the dye.

Cation-exchangers include S and CM Fast Flow, from Pharmacia.

Anion-exchangers include Pharmacia's DEAE Fast Flow and Q Fast Flow. Preferably, the matrix is Dowex-1, which is a strongly basic anion exchange resin, preferably 2% cross-linked, with a dry mesh size of 50-100. Generally, a strong anion exchanger is better than a weak exchanger.

The protein may be a serum-derived protein such as human albumin, a lipoprotein, a blood coagulation factor such as Factor VIII or Factor IX, thyroxin-binding globulin or alpha interferon. Preferably, the protein is human albumin (HA) or a mutant or fragment thereof which retains a dye-binding domain (such as is described in EP-A-322 094) or a fusion of HA or a said mutant or fragment with another protein. The aqueous liquid is suitably the direct or indirect result of exposing a fermentation medium or fractions thereof to the protein-binding compound; "indirect" in this context means that the fermentation medium, after contact with the protein-binding compound, may be treated in one or more process steps before the process of the invention is applied. By "fermentation medium" we mean the medium which results from the fermentation of an organism capable of producing the protein. The organism (which term includes cell lines) is preferably transformed or transfected to produce the protein and the protein is normally heterologous to the organism. The organism may be a bacterium (eg *E*. *coli.* or *B. subtilis*)*,* a yeast (eg *Saccharomyces cerevisiae*)*,* a non-yeast fungus (eg *Aspergillus niger),* an insect cell (eg *Spodoptera frugiperda),* a plant cell (eg a hairy root cell culture of *Atropa belladonna)* or a mammalian cell (eg Vero cells). Preferably, the organism is a yeast. Exemplary genera of yeast contemplated to be useful in the practice of the present invention are *Pichia, Saccharomyces, Kluyveromyces, Candida, Torulopsis, Hansenula, Schizosaccharomyces, Citeromyces, Pachysolen, Debaromyces, Metschunikowia, Rhodosporidium, Leucosporidium, Botryoascus, Sporidiobolus, Endomycopsis,* and the like. Preferred genera are those selected from the group consisting of *Pichia, Saccharomyces, Schizosaccharomyces, Kluyveromyces, Yarrowia* and *Hansenula,* because the ability to manipulate the DNA of these yeasts has, at present, been more highly developed than for the other genera mentioned above.

Examples of *Saccharomyces* are *Saccharomyces cerevisiae* (especially preferred), *Saccharomyces italicus* and *Saccharomyces rouxii.* Examples of Kluyveromyces are Kluyveromyces fragilis and Kluyveromyces lactis. Examples of *Hansenula* are *Hansenula polymorpha, Hansenula anomala* and *Hansenula capsulata. Yarrowia lipolytica* is an example of a suitable *Yarrowia* species, and *Schizosaccharomyces pombe* is a further suitable yeast.

The production of human albumin expressed from a gene inserted into a suitable host by recombinant DNA techniques is well known in the art and does not require discussion herein. Examples of specific prior art processes include those described in EP-A-147 198 (Delta Biotechnology), EP-A-201 239 (Delta), EP-A-60 057 (Genentech), EP-A-88 632 (Genentech), EP-A-251 744 (Delta) and EP-A-286 424 (Delta).

Similarly, processes for purifying proteins from a fermentation medium are known in the art. A good review may be found in "Protein Purification - Principles and Practice", 2nd Edition (Springer Verlag, N.Y.), especially pages 141-157.

Preferably, the aqueous liquid results from passing the fermentation medium through one or more separation (eg chromatographic) steps.

It is to be noted that, although the process of the invention is particularly well suited to separating a protein-binding compound from a protein when the protein-binding compound has been used to purify the protein from, for example, a fermentation medium or a product thereof, the process can generally be used to separate any suitable protein-binding contaminant from a protein. An advantage of the process is that it does not require binding of the protein to the resin and hence relatively large volumes of protein can be purified for a given volume of resin.

The disrupting material comprises a mixture of a salt (preferably sodium chloride or potassium chloride) and a compound to disrupt hydrophobic interactions between the protein and the protein-binding compound, for example a (preferably non-ionic) detergent, an organic solvent or, preferably, a fatty acid. Alternative disrupters of hydrophobic interactions with the protein include N-acetyltryptophan and mandelic acid, which will normally be used as their salts, for example sodium salts. The fatty acid is preferably octanoic acid but other fatty acids (preferably C₆-C₁₀ and preferably saturated) may be used. The fatty acid will usually be present in the form of its salt, for example the sodium salt. The concentrates of the salt and fatty acid may be varied to suit the particular protein and protein-binding compound in question. A salt concentration of 0.1 M to 3 M will generally be useful, preferably 0.5 to 2.0 M. A fatty acid concentration of 10 mM-100 mM is generally useful, preferably 25-60 mM, most preferably about 50 mM.

The liquid which is exposed to the ion exchange resin will usually consist largely of the buffer used to elute the protein from the column containing the protein-binding compound. The disrupting material or a component of it may then be added. For example, if the elution buffer contains 2 M NaCl in a 50 mM phosphate buffer of pH7.0, there may be no need to add further salt, and only the fatty acid is added. The pH can be altered if desired. We have found that a pH of about 7.0 is suitable, but generally any pH of above 5.0 is applicable to any fatty acid.

The pH should preferably be such that the protein-binding compound is charged; for example most polysulphonated triazine dyes are negatively charged above pH 2 to 3. It is not always necessary for the liquid to contain a buffer.

The most convenient means of exposing the mixture of the protein and protein-binding compound to the ion exchange resin and disrupting material will be to add the disrupting material to the mixture and then to pass the resulting liquid through a column of the ion exchange resin. This minimises the amounts of buffer and resin used, and the amount of protein lost. However, it is technically possible to expose the protein/protein binding compound mixture to the resin first, and then to elute the protein with a buffer containing the disrupting material. A larger column of resin will usually be needed in such an embodiment, which will then probably have to be cleaned stringently with suitable acids and solvents rather than being simply discarded.

The columns may be the conventional linear type or radial flow cartridges.

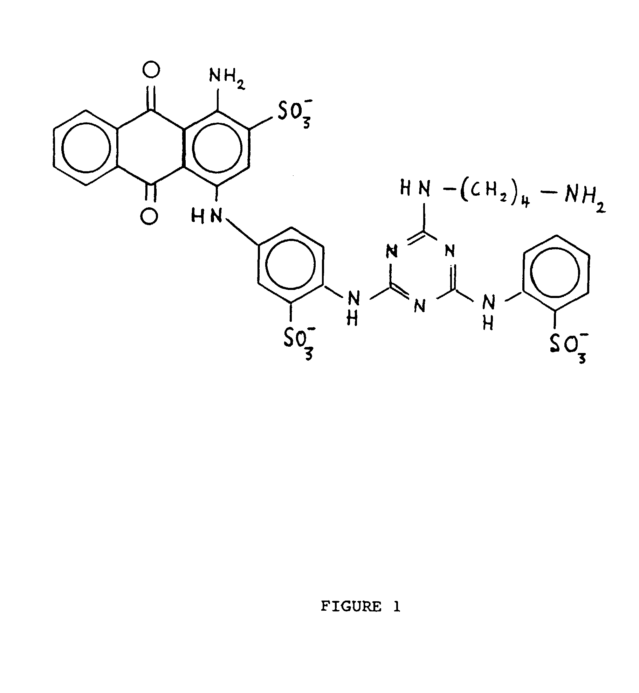

The invention will now be illustrated by way of example and with reference to Figure 1 which shows the structure of a textile dye (Cibacron Blue 3-GA) and spacer (4-amino butyl group) usable in a column to purify human albumin.

### Example 1

As a model of the product of passing an HA-containing fermentation medium through a purification column, a 3 mg.ml⁻¹ solution of human serum albumin was prepared in 2 M NaCl, 50 mM phosphate buffer pH7.0, and 21 µg.ml⁻¹ of Cibacron Blue dye covalently attached to a spacer (Fig 1) was added. The dye included a spacer used to attach the dye molecule to the matrix and also a dye synthesis intermediate. 1 M sodium octanoate, as the disrupter of hydrophobic interactions, was added to give a concentration of 50 mM. This solution (20 ml) was then passed through a 1 ml column of Dowex-1 resin (2% cross-linked; Dow Chemical Co) at a flow rate of 0.5 ml.min⁻¹. The removal of blue dye from HA was measured spectrophotometrically at 620 nm.

Under these conditions, about 97% of the blue dye bound to the resin. The unbound fraction which had passed through the column contained greater than 97% of the HA applied to the column.

### Example 2

Following the procedure of Example 1, the efficiency of dye removal from HA was assessed in the presence of buffer, 2 M NaCl, caprylate and combinations of these components. As can be seen from the results in Table 1, a combination of salt and fatty acid was much more effective than the individual components.

**TABLE 1**

| Buffer | Dye + Spacer Removal | Dye Intermediate Removal |
|---|---|---|
| | (%) | (%) |
| A 50 mM phosphate pH7.0 | 19 | N/D |
| B 50 mM phosphate + 2 M NaCl | 32 | N/D |
| C 50 mM phosphate + 50 mM caprylate | 46 | N/D |
| D Combination (B + C) | 97 | 96 |
| N/D = not determined | | |

### Example 3

The comparison of Example 2 was repeated, using Cibacron Blue 3-GA (Blue), Procion Green H-4G (Green), Procion Brown MX-5BR (Brown) and Procion Red HE-3B (Red) dyes covalently attached to a spacer. The results are shown in Table 2.

**TABLE 2**

| HSA/Dye + Spacer Separation (% removal) | | | | |
|---|---|---|---|---|
| Buffer | Blue | Green | Brown | Red |
| A | 19 | 11 | 52 | 33 |
| B | 32 | 41 | 93 | 57 |
| C | 46 | 45 | 90 | 83 |
| D | 97 | 65 | 89 | 92 |
| A-D as Table 1 | | | | |

### Example 4

The experiment of Example 2 was repeated with different proteins. The results are shown in Table 3. Alkaline phosphatase was mixed with blue or red dyes.

**TABLE 3**

| Protein/Dye + Spacer Separation (% removal) | | | | | | |
|---|---|---|---|---|---|---|
| Buffer | HSA | LACTOFERRIN | ADH | GK | AP/Blue | AP/Red |
| A | 19 | 66 | N/D | N/D | 52 | N/D |
| B | 32 | N/D | 42 | 73 | 80 | 63 |
| C | 46 | 67 | 72 | 96 | 80 | 71 |
| D | 97 | 81 | 94 | 91 | 84 | 92 |
| A-D as Table 1 GK = glycerokinase ADH = alcohol dehydrogenase AP = alkaline phosphatase N/D = not determined | | | | | | |

## Claims

1. A process for removing some or all of a protein-binding compound from an aqueous liquid containing the protein-binding compound bound to a protein, the process comprising the steps of (1) exposing the liquid to a disrupting material comprising a mixture of a salt and a compound which disrupts hydrophobic interactions between the protein and the protein-binding compound, (2) exposing the liquid from step (1) to an ion exchange resin to bind the protein-binding compound to the resin and (3) separating the resin to yield the protein, wherein either (i) the concentration of the salt is at least 0.5M, or (ii) the protein-binding compound is a synthetic textile dye or a protein-binding intermediate or derivative thereof, or (iii) the protein is human albumin.

2. A process for removing some or all of a protein-binding compound from an aqueous liquid containing the protein-binding compound bound to a protein, the process comprising the steps of (1) exposing the liquid to an ion exchange resin to bind the protein-binding compound to the resin, (2) contacting the thus-bound protein-binding compound with a disrupting material comprising a mixture of a salt and a compound which disrupts hydrophobic interactions between the protein and the protein-binding compound, and (3) separating the resin to yield the protein, wherein either (i) the concentration of the salt is at least 0.5M, or (ii) the protein-binding compound is a synthetic textile dye or a protein-binding intermediate or derivative thereof, or (iii) the protein is human albumin.

3. A process according to Claim 1 in which the step involving the disrupting material and the step of binding the protein-binding compound to the resin are carried out simultaneously or such that they overlap.

4. A process according to any one of the preceding claims wherein the protein-binding compound is a synthetic textile dye or an intermediate or derivative thereof.

5. A process according to Claim 4 wherein the protein-binding compound is a triazine dye or an intermediate or derivative thereof.

6. A process according to any one of the preceding claims wherein the ion-exchange resin is a strongly basic anion exchange resin.

7. A process according to any one of the preceding claims wherein the protein is human albumin (HA) or a mutant or fragment thereof which mutant or fragment retains a dye-binding domain, or a fusion of HA or a said mutant or fragment with another protein.

8. A process according to any one of the preceding claims wherein the compound to disrupt hydrophobic interactions is a fatty acid or a salt thereof.

9. A process for preparing a protein comprising:-
(i) fermenting an organism capable of producing the protein such that the protein is produced,
(ii) exposing the protein-containing fermentation medium obtained from step (i), or a liquid derived therefrom and containing the said protein, to an immobilised protein-binding compound to bind the protein thereto,
(iii) separating the protein from the immobilised protein-binding compound to yield the aqueous liquid of Claim 1 or Claim 2, and
(iv) subjecting the aqueous liquid from step (iii) to a process according to any one of Claims 1 to 8 to remove at least some of any protein-binding compound associated with the protein.

## Patentansprüche

1. Verfahren zum Entfernen von etwas oder der Gesamtmenge einer proteinbindenden Verbindung aus einer wäßrigen Flüssigkeit, die die an ein Protein gebundene, proteinbindende Verbindung enthält, durch
(1) Einwirkenlassen eines Aufbrechmaterials in Form eines Gemischs aus einem Salz und einer Verbindung, die hydrophobe Wechselwirkungen zwischen dem Protein und der proteinbindenden Verbindung aufbricht, auf die Flüssigkeit,
(2) Behandeln der Flüssigkeit aus Stufe (1) mit einem Ionenaustauschharz zur Bindung der proteinbindenden Verbindung an das Harz und
(3) Abtrennen des Harzes zur Gewinnung des Proteins, wobei entweder (I) die Konzentration an dem Salz mindestens 0,5 M beträgt oder (II) die proteinbindende Verbindung aus einem synthetischen Textilfarbstoff oder einem proteinbindenden Zwischenprodukt oder Derivat hiervon besteht oder (III) das Protein aus Humanalbumin besteht.

2. Verfahren zum Entfernen von etwas oder der Gesamtmenge einer proteinbindenden Verbindung aus einer wäßrigen Flüssigkeit, die die an ein Protein gebundene, proteinbindende Verbindung enthält, durch
(1) Behandeln der Flüssigkeit mit einem Ionenaustauschharz zur Bindung der proteinbindenden Verbindung an das Harz,
(2) Kontaktieren der derart gebundenen, proteinbindenden Verbindung mit einem Aufbrechmaterial in Form eines Gemischs aus einem Salz und einer Verbindung, die hydrophobe Wechselwirkungen zwischen dem Protein und der proteinbindenden Verbindung aufbricht, und
(3) Abtrennen des Harzes zur Gewinnung des Proteins, wobei entweder (I) die Konzentration an dem Salz mindestens 0,5 M beträgt oder (II) die proteinbindende Verbindung aus einem synthetischen Textilfarbstoff oder einem proteinbindenden Zwischenprodukt oder Derivat hiervon besteht oder (III) das Protein aus Humanalbumin besteht.

3. Verfahren nach Anspruch 1, bei welchem die Stufe, in der das Aufbrechmaterial beteiligt ist und die Stufe, in der die proteinbindende Verbindung an das Harz gebunden wird, gleichzeitig oder überlappend durchgeführt werden.

4. Verfahren nach einem der vorhergehenden Ansprüche, wobei die proteinbindende Verbindung aus einem synthetischen Textilfarbstoff oder einem Zwischenprodukt oder einem Derivat hiervon besteht.

5. Verfahren nach Anspruch 4, wobei die proteinbindende Verbindung aus einem Triazinfarbstoff oder einem Zwischenprodukt oder einem Derivat hiervon besteht.

6. Verfahren nach einem der vorhergehenden Ansprüche, wobei es sich bei dem Ionenaustauschharz um ein stark basisches Anionenaustauschharz handelt.

7. Verfahren nach einem der vorhergehenden Ansprüche, wobei das Protein aus Humanalbumin (HA) oder einer Mutante oder einem Bruchstück hiervon, bei der (dem) jeweils eine farbstoffbindende Domäne erhalten geblieben ist, oder einem Fusionsprodukt von HA oder der Mutante oder des Bruchstücks mit einem anderen Protein besteht.

8. Verfahren nach einem der vorhergehenden Ansprüche, wobei es sich bei der Verbindung zum Aufbrechen hydrophober Wechselwirkungen um eine Fettsäure oder ein Salz derselben handelt.

9. Verfahren zur Herstellung eines Proteins durch
(1) Fermentieren eines Organismus mit der Fähigkeit zur Herstellung des Proteins dergestalt, daß das Protein produziert wird,
(2) Einwirkenlassen einer immobilisierten proteinbindenden Verbindung auf das in Stufe (1) erhaltene proteinhaltige Fermentationsmedium oder eine aus diesem herrührende und das Protein enthaltende Flüssigkeit zur Bindung des Proteins an die betreffende Verbindung,
(3) Abtrennen des Proteins von der immobilisierten proteinbindenden Verbindung zur Gewinnung der wäßrigen Flüssigkeit nach Anspruch 1 oder Anspruch 2 und
(4) Behandeln der wäßrigen Flüssigkeit aus Stufe (3) im Rahmen eines Verfahrens nach einem der Ansprüche 1 bis 8 zur Entfernung mindestens eines Teils irgendeiner mit dem Protein vergesellschafteten proteinbindenden Verbindung.

## Revendications

1. Procédé pour enlever tout ou partie d'un composé fixant une protéine à partir d'un liquide aqueux contenant le composé fixant une protéine, lié à un protéine, le procédé comprenant les étapes consistant à (1) exposer le liquide à une matière disruptive comprenant un mélange d'un sel et d'un composé qui brise les interactions hydrophobes entre la protéine et le composé fixant une protéine, (2) exposer le liquide de l'étape (1) à une résine échangeuse d'ions pour lier le composé fixant une protéine à la résine et (3) à séparer la résine pour donner la protéine, dans lequel soit (i) la concentration du sel est d'au moins 0,5 M, soit (ii) le composé fixant une protéine est un colorant textile synthétique ou un produit intermédiaire fixant une protéine ou un dérivé de celui-ci, soit (ii) la protéine est de l'albumine humaine.

2. Procédé pour enlever tout ou partie d'un composé fixant une protéine à partir d'un liquide aqueux contenant le composé fixant une protéine, lié à un protéine, le procédé comprenant les étapes consistant à (1) exposer le liquide à une résine échangeuse d'ions pour lier le composé fixant une protéine à la résine, (2) mettre le composé fixant une protéine ainsi lié en contact avec une matière disruptive comprenant un mélange d'un sel et d'un composé qui brise les interactions hydrophobes entre la protéine et le composé fixant une protéine et (3) à séparer la résine pour donner la protéine, dans lequel soit (i) la concentration du sel est d'au moins 0,5 M, soit (ii) le composé fixant une protéine est un colorant textile synthétique ou un produit intermédiaire fixant une protéine ou un dérivé de celui-ci, soit (ii) la protéine est de l'albumine humaine.

3. Procédé selon la revendication 1, dans lequel l'étape faisant intervenir la matière disruptive et l'étape de liaison du composé fixant une protéine à la résine sont effectuées simultanément ou de sorte qu'elles se chevauchent.

4. Procédé selon l'une quelconque des revendications précédentes, dans lequel le composé fixant une protéine est un colorant textile synthétique ou un produit intermédiaire ou dérivé de celui-ci.

5. Procédé selon la revendication 4, dans lequel le composé fixant une protéine est un colorant triazine ou un produit intermédiaire ou dérivé de celui-ci.

6. Procédé selon l'une quelconque des revendications précédentes, dans lequel la résine échangeuse d'ions est une résine échangeuse d'anions fortement basique.

7. Procédé selon l'une quelconque des revendications précédentes, dans lequel la protéine est de l'albumine humaine (HA) ou un mutant ou fragment de celle-ci, ledit mutant ou fragment retenant un domaine de liaison à un colorant, ou une fusion de HA ou dudit mutant ou fragment avec une autre protéine.

8. Procédé selon l'une quelconque des revendications précédentes, dans lequel le composé pour briser les interactions hydrophobes est un acide gras ou un sel de celui-ci.

9. Procédé pour préparer une protéine, comprenant les étapes consistant à :
(i) faire fermenter un organisme capable de produire la protéine, de sorte que la protéine est produite,
(ii) exposer le milieu de fermentation contenant la protéine obtenu dans l'étape (i), ou un liquide dérivé de celui-ci et contenant ladite protéine, à un composé fixant une protéine immobilisé pour lier la protéine à celui-ci,
(iii) séparer la protéine du composé fixant une protéine immobilisé pour donner le liquide aqueux selon la revendication 1 ou la revendication 2, et à
(iv) soumettre le liquide aqueux de l'étape (iii) à un procédé selon l'une quelconque des revendications 1 à 8 pour enlever au moins une partie de tout composé fixant une protéine associé avec la protéine.
